(19) Europäisches Patentamt
European Patent Office
Office européen des brevets

(11) EP 1 111 533 A2

(12) **EUROPEAN PATENT APPLICATION**

(43) Date of publication:
**27.06.2001 Bulletin 2001/26**

(51) Int Cl.[7]: **G06F 19/00**

(21) Application number: **00310926.1**

(22) Date of filing: **08.12.2000**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **15.12.1999 US 170917 P**

(71) Applicant: **Pfizer Products Inc.**
**Groton, Connecticut 06340 (US)**

(72) Inventor: **Potter, David Martin**
**Groton, Connecticut 06340 (US)**

(74) Representative: **Eddowes, Simon et al**
**Urquhart-Dykes & Lord,**
**30 Welbeck Street**
**London W1G 8ER (GB)**

(54) **Logistic regression trees for drug analysis**

(57) An overall system and method for filtering out compounds with potentially poor pharmacokinetic properties, such as unfavorable metabolism or low solubility. The filtering is effected without experimental testing but from consideration as drug candidates or validation or rejection of compounds as drug candidates. This filtering is achieved by comparison of the subject compounds with statistically significant features in a database of already determined acceptable and non-acceptable compounds, by means of a logistic regression tree analysis. A logistic regression tree model and inquiry system is adapted to determined statistically common characteristics (referred to as candidate predictor variables) to structurally compare candidate compounds with the requisite characteristics such as ADME of acceptable compounds.

**FIG. 6**

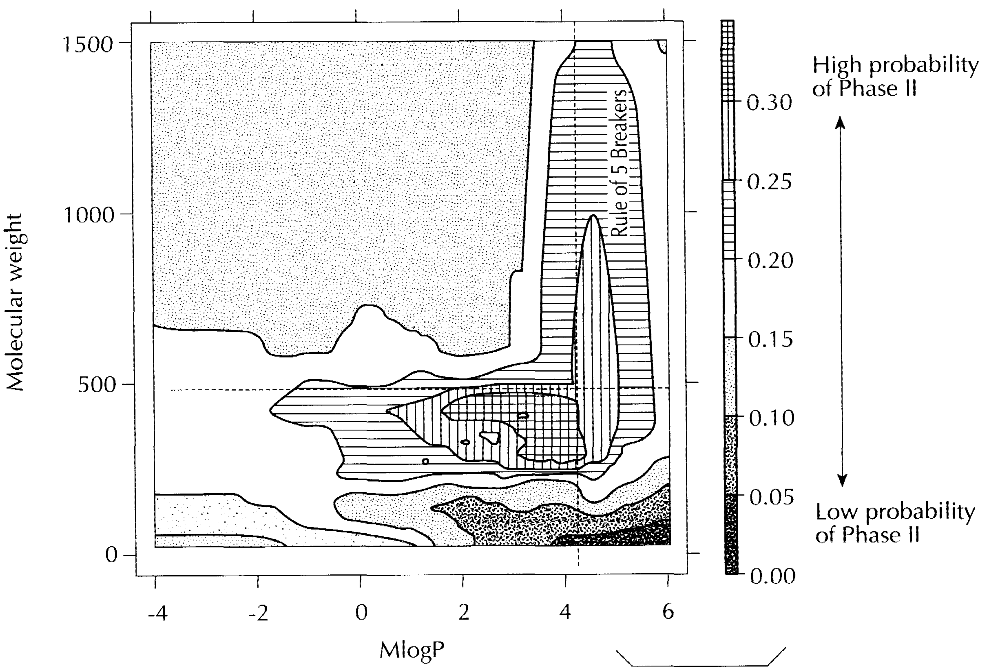


EP 1 111 533 A2

## Description

This invention relates to logical decisions methods and systems, for aid in assessing or predicting the viability of selected compounds for drug possibilities (i.e., having favorable pharmacokinetic characteristics such as good oral absorption). The invention also relates to such methods and systems for assessing the worth of additional research and testing, without experimentation or testing, but only based on compound structures, and particularly relates to methods using pre-determined or pre-set parameters indicative of general trends and utilities of classes of compounds.

## Background of the Invention

In a drug discovery setting there is an interest in determining, or at least predicting, various basic pharmacokinetic properties (e.g., ADME-absorption, distribution, metabolism, and excretion) from the molecular structure of a subject compound alone. This determination or, more properly, an educated prediction, serves to focus efforts on compounds which have a more viable possibility of development to actual drugs. To this end a determination system has been developed, as set forth in detail in *Experimental and computational approaches to estimate solubility and permeability in drug discovery and development setting,* by Christopher A. Lipinski et al. (Advanced Drug Delivery Reviews 23, pp 3-25 (1997)) known colloquially as the "Rule of 5", which provides a rough guide, related to the number "5", for filtering out compounds with potentially poor pharmacokinetic properties (including the ADME properties), such as poor oral absorption, without the necessity for conducting actual tests and experiments. This approach can improve the design of virtual libraries of compounds for screens against a particular biological target. More precisely, libraries comprising compounds likely to have good ADME properties should reduce the chance that a lead compound will run into pharmacokinetic difficulties during later studies. The non-experimental filtering is of particular importance as pharmaceutical companies move toward large-scale screening research procedures.

The "Rule of 5" components, which indicate that poor absorption/permeation are more likely, are: a) more than 5 H-bond donors (sum of OH's, NH's), b) the molecular weight is over 500, c) the ClogP is over 5 (or MlogP is over 4.15), and d) more than 10 H-bond acceptors (sum of N's, O's). When at least two of such criteria are satisfied then there is a question about the viability of the compound for further study as a drug candidate. "ClogP" and "MlogP" are common measurements of lipophilicity, an important factor in drug evaluation. These are however rigid factors and other characteristics may be of overriding importance and there have been instances of useful drugs which do not meet the Rule of 5 criteria.

## Summary of the Invention

It is an object of the present invention to provide a system and method which provides a basis for more accurately determining (or providing educated estimates) validity of candidate compounds as being useful drugs, based on structure alone, without actual experimentation or testing.

It is a further object of the present invention to provide a system and method which utilizes logistic regression tree analysis, with inquiry parameters based on data culled from a sufficient, and normally extensive, data base of previously determined viable compounds for candidate drug research (with an indicated history of actual use as a drug), as well as compounds which were determined as not being viable. The compounds of the data base serve as a measure, based on the inquiry analysis, for predicting further utility in other compounds with similar defined characteristics, such as of structure, components and molecular weight.

Generally the present invention comprises an overall system and method for filtering out compounds with potentially poor pharmacokinetic properties such as poor oral absorption, without experimental testing. This filtering is achieved by comparison of the features of the subject compound with both features determined to be significantly associated with a good pharmacokinetic profile and with features associated with a bad pharmacokinetic profile, the latter being determined using a database of both already determined acceptable and non-acceptable compounds in order to build a model that distinguishes the two.

In accordance with the system and method of the present invention, a system (preferably computerized because of the number of compounds and variables which may be involved) is used and the method is implemented with a data base of known and characterized compounds according to the following steps:

a) setting up a data base of compounds which have been previously experimentally determined to have good pharmacokinetic properties and compounds which have been previously experimentally determined to have bad pharmacokinetic properties; to provide a statistical basis for various common structural characteristics, components and determined properties; wherein each compound entry for the data base contains at least the structural configurations for the entry compound and whether the compound was actually used in clinical trials;
b) adapting a logistic regression tree model and inquiry system to determined statistically common characteristics

of the compounds in said data base, as candidate predictor variables, to structurally compare candidate compounds with characteristics of acceptable and not acceptable compounds in said data base;

c) applying the logistic regression tree model to compounds in said data base to provide a fit of the candidate compounds with candidate predictor variables;

d) using the logistic regression tree model to recursively search the candidate compounds with the predictor variables, to yield the best overall fit of the candidate compounds with the predictor variable; and

e) using this best overall fit in final determinations for viability of candidate compounds as candidates for testing as drugs.

More specifically, the method comprises the steps wherein:

a) the computerized data base is initially set up in memory or accessible data storage of compounds which have been previously determined to have the requisite ADME characteristics (whether or not a compound has made it to Phase II clinical trials is acceptable as being used as a proxy for "good pharmacokinetic/ADME profile" with it again being noted that the database also contains compounds that do not have a good PK/ADME profile), to provide a statistical basis for various common structural characteristics and determined properties. The extent of the data base, sufficient to provide a statistically significant result, is dependent on the properties being used as parameters. Accordingly, a data base of several hundred compounds may be sufficient for one analysis (e.g., such as where the common characteristics are confined to a small number of compounds) while another data base may require thousands of representative compounds to provide compatible and significant comparisons with similar compounds. An ad hoc determination is thus required for each application with respect to minimization of data base comparisons without compromising the validity of any analysis. The data base should only comprise compounds having structural and/or other characteristics in common with the compound under investigation or a determinable nexus such as lack or presence of attributes in compounds having bad pharmacokinetic characteristics.

Each compound entry for the data base should contain at least the structural configurations (or an enumeration of components on an atomic or molecular level) for the entry compounds, for proper identification of compounds, and cross checks for computing the physical-chemical variables (e.g., molecular weight). Preferably there are also other compound identification means such as CAS registration numbers, and approved name, for additional cross checking and verification of analysis; calculated physical-chemical properties thought to be related to pharmacokinetic profile (e.g., molecular weight, measures of lipophilicity, hydrogen bonding features); optionally, therapy use and mechanism (if known); and whether a compound was actually used as a drug or whether the compound progressed to Phase II clinical trials as reflected by presence of ISAN or UNN name.

b) A logistic regression tree model and inquiry system is adapted to determined statistically common characteristics (referred to as candidate predictor variables) to structurally compare candidate compounds with the requisite ADME characteristics of acceptable and non-acceptable compounds. This differs from prior art determinations, such as the Rule of 5, which are based on rigidly preset parameters generally, but not specifically, derived from prior compound experience. Logistic regression trees are applicable in a setting with a binary outcome such as determination of compounds for drug viability (either yes or no) and a set of candidate explanatory variables (either continuous or categorical). Logistic regression trees are useful in identifying interactions among the explanatory variables (i.e., where the effect of an explanatory variable depends on the value of another explanatory variable), and nonlinear relationships (i.e., where the effect of an explanatory variable on the binary response is not strictly increasing or decreasing). In addition, a logistic regression tree model can be used to predict the probability of the binary response based on values of the explanatory variables.

Specifically, in accordance with the present invention, a tree-based statistical method is utilized which imbeds logistic regression models within a recursive partitioning framework. More precisely, logistic regression is a traditional statistical approach to modeling the probability of a binary response as a function of one or more explanatory variables. Tree-based methods, also known as recursive partitioning methods, model a binary response by repeatedly splitting the data into subgroups, based on values of the predictors, such that the resulting subgroups are homogeneous with respect to the binary response. Combining the two approaches, logistic regression trees repeatedly split the data into subgroups, based on values of the predictors, such that the logistic regression models in the subgroups provide a good fit to the data.

c) Applying the logistic regression tree model to candidate compounds to provide a fit of the compounds with idealized regressor or predictor variables of the compounds of the data base. Given a binary outcome (accept or reject) and a set of candidate predictor variables (culled from the data base), this *logistic regression tree* approach recursively searches for the predictors, their split points, and logistic regression models that together yield the best overall fit.

d) Using this best overall fit in final determinations of compound (or series of compounds) viability as a drug or drug candidate.

The above and other objects, features and advantages of the present invention will become more evident from the following discussion and drawings in which:

**SHORT DESCRIPTION OF THE DRAWINGS**

Figures 1-5 represent a progressive graphing of likelihood of compounds becoming candidates for viable drugs against MlogP, based on successive logistic regressive tree analysis and based on molecular weight predictor factors; and
Figure 6 is an overall area graph of molecular versus MlogP showing degree of drug viability probability.

<u>**Detailed Description of the Invention**</u>

The viability of the method of the present invention was tested by using a database of physico-chemical properties for 40,000 structurally defined literature compounds, many of which were used as actual drugs. In a comparison study, a logistic regression tree analysis was used to identify a set of compounds that have a high probability of progression to advanced clinical drug trials in spite of having a physico-chemical property profile that would suggest poor oral absorption in accordance with prior art determination parameters such as higher molecular weight being more indicative of poorer absorption. A particular example of the compounds was dexniguldipine, with a structure:

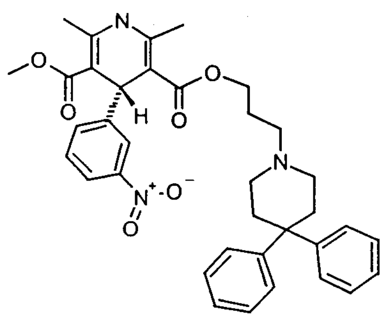

Chiral

The data base contained compounds (both acceptable and non-acceptable) with one or more structurally overlapping features in common with the above compound and the other compounds being analyzed for viability of the present method.

A closer look at the compounds exhibited the disparity between viability according to evaluation based on the method of the present invention and evaluation based on analysis with methods of the prior art. It revealed that these compounds may act as inhibitors of the P-glycoprotein transporter, which acts as an efflux pump to reduce intestinal absorption of foreign molecules. With the compounds being able to reduce efflux pump effect, the time the compound (as a foreign molecule) is effectively at a site (without being expulsed) with increased absorption is extended. Thus, a chemically less effective compound per se (as determined such as by Rule of 5 criteria) is determined to be more effective as a result of enhanced take-up based on an efflux pump effect.

In accordance with the method of the present invention the regression tree analysis is effected by the following steps:

1. Start with all compounds to be analyzed for viability;
2. Choose a single regressor variable (e.g., MlogP) for selective basic analysis;
3. Fit the logistic regression model to a data base of known compounds (both viable and not viable) according to the formula;

$$\log\left(\frac{\text{Pr(Phase II)}}{\text{1-Pr(PhaseII)}}\right) = \beta_0 + \beta_1 \text{MlogP}$$

wherein "Pr(Phase II)" denotes the probability "p" of viability for a given compound; $\beta_o$ represents the intercept of the model and $\beta_1$ represents the effect on log(p/(1-p)) of a one unit increase in MlogP. The logistic regression

model is estimated using maximum likelihood estimation, via a Newton-Raphson algorithm.

4. For each predictor variable *X*, and for every split point *c* on *X*, separate compounds into 2 sets, $S_{LEFT}$ & SRIGHT respectively:

$S_{LEFT}$: all compounds for which $X < c$
$S_{RIGHT}$: all compounds for which $X \geq c$.

5. In each set, fit a logistic regression model:

$$S_{LEFT}: \log \left( \frac{\Pr(\text{Phase II})}{1-\Pr(\text{PhaseII})} \right) = \beta_{0,left} + \beta_{1,left}\text{MlogP}$$

$$S_{RIGHT}: \log \left( \frac{\Pr(\text{Phase II})}{1-\Pr(\text{PhaseII})} \right) = \beta_{0,right} + \beta_{1,right}\text{MlogP}$$

6. Choose the variable *X** and split point *c** that yield the best fit as measured by the deviance (a goodness-of-fit measure).
7. Split the compounds into 2 sets based on *X** and *c**.
8. Recursively repeat steps 3-7 for $S_{LEFT}$ and $S_{RIGHT}$.
9. Select tree size by cross-validation (CV) or use of external test set.
10. Repeat entire process for other regressors.
11. Choose tree with minimum CV or test set deviance for selection of the most viable candidate compounds.

## DETAILED DESCRIPTION OF THE DRAWINGS AND THE PREFERRED EMBODIMENTS

The relationship between successful progression to viable drug candidacy and the Rule of 5 variables was tested with the use of logistic regression trees, as described, utilizing a 40,000 compound database.

The logistic regression tree suggests that the effect of MlogP on successful progression to drug viability candidacy (Phase II) *depends* on the molecular weight of a compound, and this is shown as the 1st split from Figure 1 (all the compounds in the data base showing a general increase of Phase II success with increasing MlogP up to about MlogP=4) to Figures 2 and 3, with a cutoff of 228 as the molecular weight.

In the Figures 1-5, in each node of the tree the probability of a compound progressing to Phase II clinical trials was plotted versus the compound's MlogP value. Because the Phase II outcome is binary (1=yes, 0=no), in addition to the logistic regression fit, a smoothed estimate of the binary response probability (i.e., a weighted average of the 1's and 0's for a narrow range of MlogP values) was also plotted (as shown in dotted line) to provide an overall estimate of the relationship without reference to any particular model.

The compounds in Figure 2 show a decline in viability whereas the compounds in Figure 3 show a trend similar to all compounds as shown in Figure 1, with a decline when MlogP is above about 4. This decline is consistent with the Rule of 5.

Figure 2 shows compounds with a molecular weight of less than 228, for which the effect of MlogP is to decrease the chances of the compound progressing to Phase II (or higher viability as a drug candidate). This decline is hypothesized to be the result of smaller compounds permeating membranes through a sieving mechanism where increased lipophilicity (i.e., MlogP) which may serve only to increase toxicities. This factor is only evident from an analysis that separates the compounds based on molecular weight (note that the Rule of 5 gives an upper molecular weight for non-viability but not a lower weight). No further separation of the compounds in Figure 2 yields an appreciable improvement in model fit, and hence this group of compounds is not further subdivided.

The logistic regression tree algorithm is then applied to the compounds with weights above 228 to provide the graph of Figure 3, with a traditional parabolic relationship of MlogP to absorption. The compounds are then split again according to a molecular weight of 497 (about the upper molecular weight limit for viability according to the Rule of 5), to provide the graphs of Figures 4 (molecular weight below 497) and Figure 5 (molecular weight above 497).

As shown in Figure 4, for compounds < ~500 g (but above 228 g), the probability of Phase II drops as MlogP values exceed the Rule of 5 cutoff. However, for compounds > ~500 g, increasing MlogP above the Rule of 5 cutoff does not decrease the chance of progression to Phase II, a totally unexpected result. Compounds which do not fit within the Rule of 5 parameters but which are indicated being viable according the logistic regression tree analysis of the present invention, and which made it to Phase II viability, include many compounds which block efflux pumps (e.

g., P-glycoprotein pump). Compounds in this category include dihydropyridines; verapamil analogs; macrolides; HIV protease inhibitors; and Prodrugs. These compounds are shown in Figure 6, in the upper right hand section with MlogP above 4 and molecular weights above 500.

Since blocking efflux pumps can be desirable or undesirable, drug candidates (and failures) may be experimentally profiled such as by using a competitive assay as standards to set up filters for different benefit/risk scenarios.

## Claims

**1.** A method for filtering out candidate compounds with potentially good or poor physiological properties with respect to further testing as candidates for drugs, without experimental testing, said method comprising the steps of:

a) setting up a data base of compounds which have been previously experimentally determined to have good pharmacokinetic properties and compounds which have been previously experimentally determined to have poor pharmacokinetic properties; to provide a statistical basis for various common structural characteristics and determined properties; wherein each compound entry for the data base contains at least the structural configurations for the entry compound and whether the compound was actually used in clinical trials;
b) adapting a logistic regression tree model and inquiry system to determined statistically common characteristics of the compounds in said data base, as candidate predictor variables, to structurally compare candidate compounds with characteristics of acceptable and not acceptable compounds in said data base;
c) applying the logistic regression tree model to compounds in said data base to provide a fit of the candidate compounds with candidate predictor variables;
d) using the logistic regression tree model to recursively search the candidate compounds with the predictor variables, to yield the best overall fit of the candidate compounds with the predictor variable; and
e) using this best overall fit in final determinations for viability of candidate compounds as candidates for testing as drugs.

**2.** The method of claim 1 wherein adapting, applying and using the logistic regression tree model of steps b-d is effected by the following steps:

a) choosing a single regressor variable for selective basic analysis as a logistic regression model;
b) fitting the logistic regression model to the data base of known acceptable and non-acceptable compounds according to the formula;

$$\log\left(\frac{\Pr(\text{Phase II})}{1-\Pr(\text{PhaseII})}\right) = \beta_0 + \beta_1 \text{MlogP}$$

wherein "Pr(Phase II)" denotes the probability "p" of viability for a given compound; $\beta_o$ represents the intercept of the model and $\beta_1$ represents the effect on log(p/(1-p) of a one unit increase in MlogP;
c) for each predictor variable $X$, and for every split point $c$ on $X$, separating compounds into two sets, $S_{\text{LEFT}}$ & $S_{\text{RIGHT}}$ respectively:

$S_{\text{LEFT}}$: all compounds for which $X \leq c$
$S_{\text{RIGHT}}$: all compounds for which $X \geq c$;

d) in each set, fitting a logistic regression model:

$$S_{\text{LEFT}}: \log\left(\frac{\Pr(\text{Phase II})}{1-\Pr(\text{PhaseII})}\right) = \beta_{0,\text{left}} + \beta_{1,\text{left}} \text{MlogP}$$

$$S_{\text{RIGHT}}: \log\left(\frac{\Pr(\text{Phase II})}{1-\Pr(\text{PhaseII})}\right) = \beta_{0,\text{right}} + \beta_{1,\text{right}} \text{MlogP};$$

e) choosing the variable $X^*$ and split point $c^*$ that yield the best fit as measured by the deviance, as a goodness-of-fit measure;
f) splitting the compounds into two sets based on $X^*$ and $c^*$;
g) recursively repeating steps 2-6 for SLEFT and SRIGHT;
h) selecting tree size by any of cross-validation (CV) and of an external test set;
i) repeating the entire process for other regressors; and
j) choosing a tree with minimum CV or test set deviance for selection of the most viable candidate compounds.

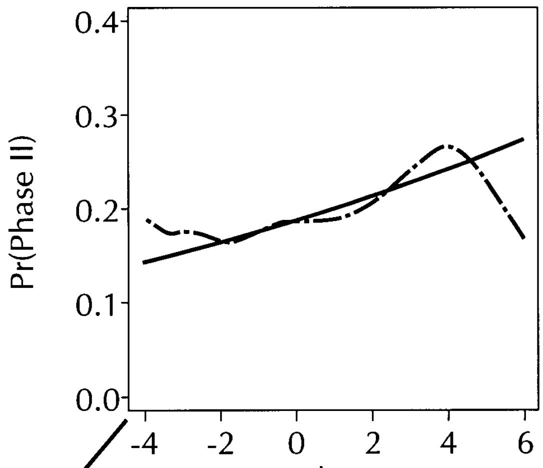


FIG. 1

Molecular weight
<=228

Molecular weight
>228

FIG. 2

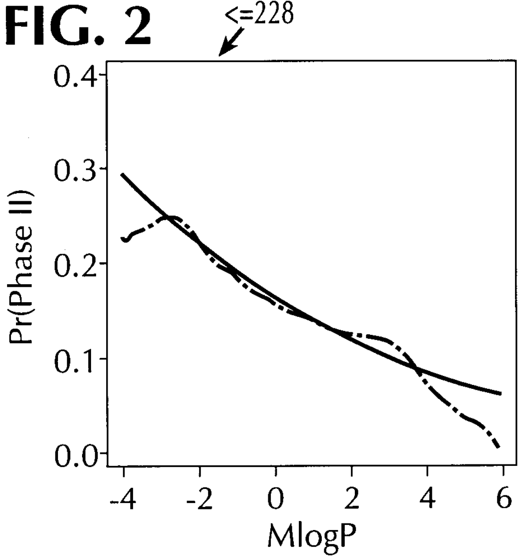


FIG. 3

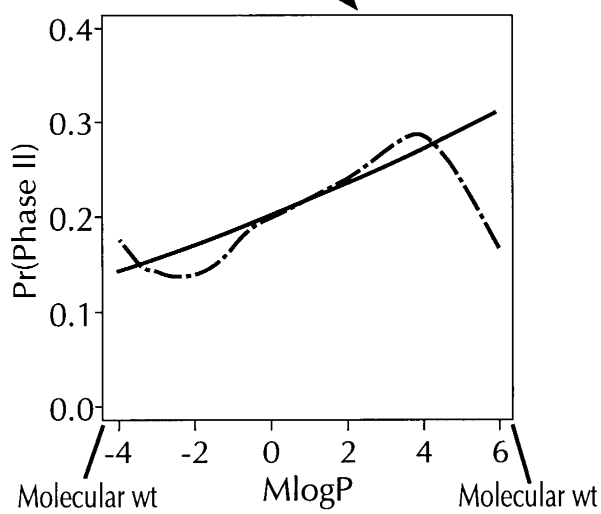


Molecular wt
<=497

Molecular wt
>497

FIG. 4

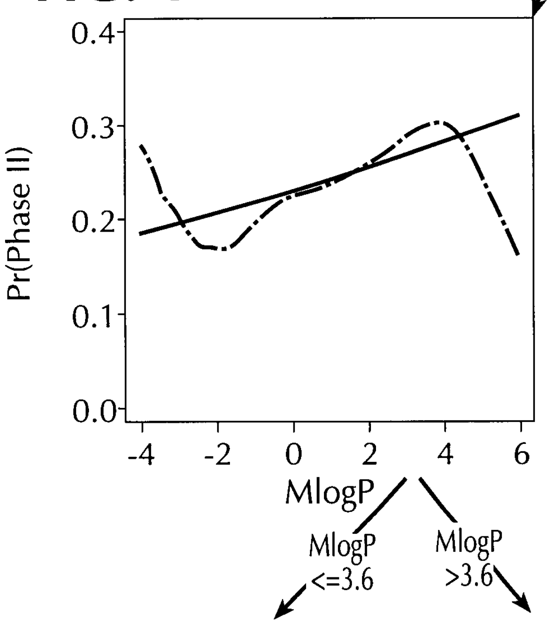


FIG. 5

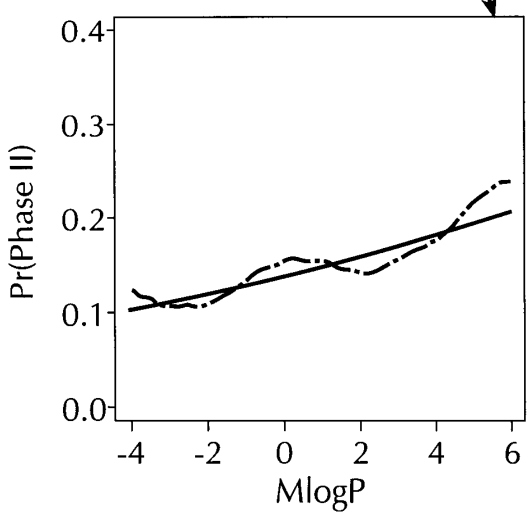


MlogP
<=3.6

MlogP
>3.6

FIG. 6

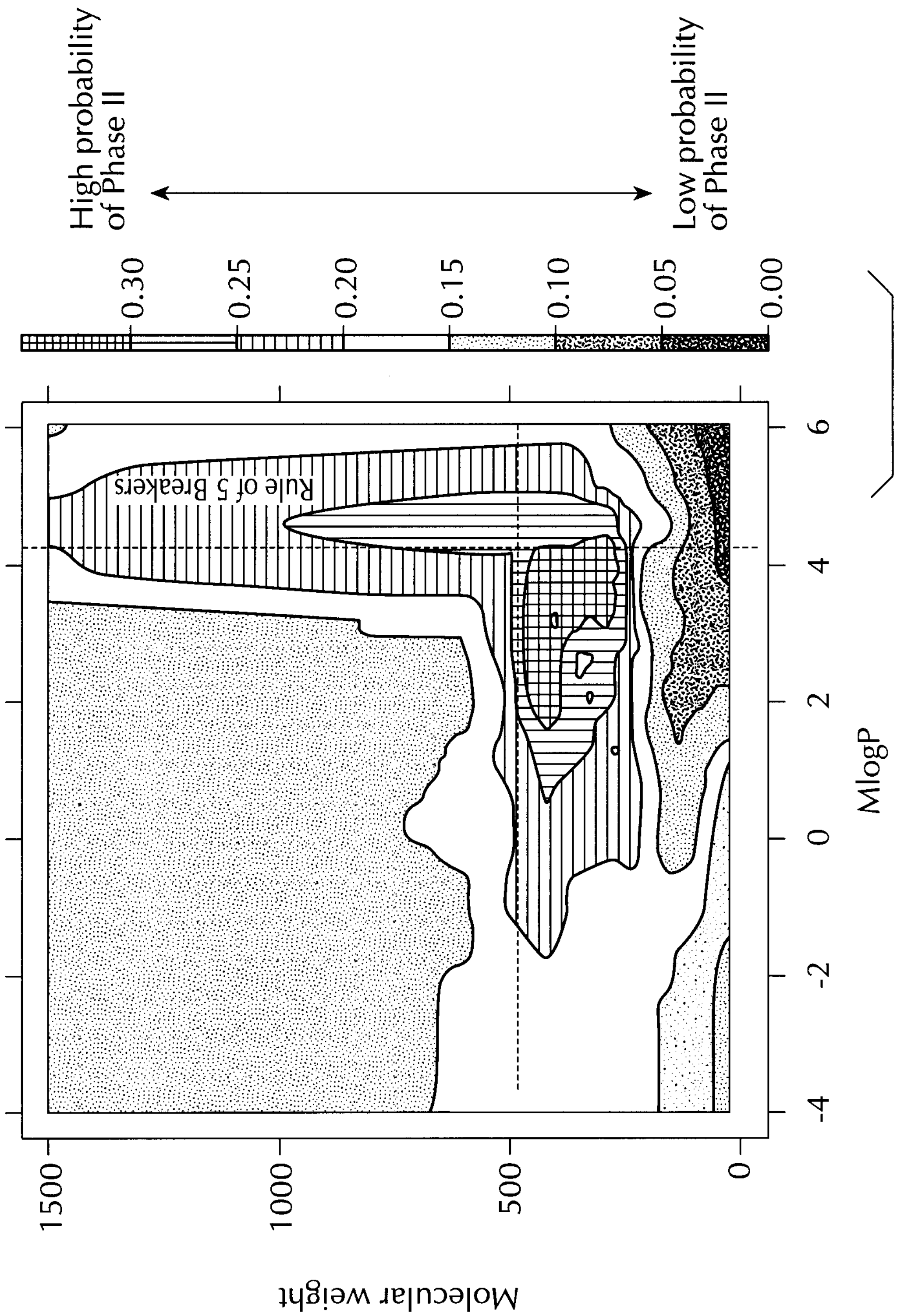
High probability of Phase II
Low probability of Phase II
0.30
0.25
0.20
0.15
0.10
0.05
0.00
Rule of 5 Breakers
Molecular weight
1500
1000
500
0
MlogP
-4
-2
0
2
4
6